# EUROPEAN PATENT APPLICATION

(11) **EP 4 309 689 A2**
(43) Date of publication of application: **24.01.2024**
(21) Application number: 23201191.6
(22) Date of filing: 31.01.2018
(51) Int. Cl.: A61L 31/14

(54) **SELF-FIXING MESH ENDOPROSTHESIS BASED ON TITANIUM THREAD AND BIORESORBABLE POLYMERS**

(62) Divisional of application: 18903554.6
(71) Applicant: Titanium Textiles AG, 18182 Rostock-Bentwisch (DE)
(72) Inventor: Kazantsev, Anton Anatolevich, Ekaterinburg 620062 (RU); Yusupov, Ajrat Auhatovich, Verhnyaya Pyshma, Sverdlovskaya obl., 624090 (RU); Alehin, Alexandr Ivanovich, Moscow 117639 (RU); Zavaruev, Vladimir Andreevich, Moscow 111621 (RU)
(74) Representative: Hoffmann Eitle

(57) **Abstract**

The invention relates to the field of medicine and medical technology and is aimed at improving technical mesh endoprostheses for hemioplasty. A self-fixing mesh endoprosthesis comprises or consists of two layers, upper and lower, with a layer of mesh based on titanium thread on the upper side, and a layer of bioresorbable polymers on the lower side, wherein the surface of the titanium thread is made in relief. The technical result consists in increasing the efficiency of fixing the endoprosthesis in the operating field, increasing the plasticity and reducing the risk of endoprosthesis threads breakage, increasing the simplicity and strength of fixation, ensuring the uniformity of fixing the endoprosthesis with the entire surface thereof to the tissues of the operating wound, reducing the duration of surgery, and, as a consequence, reducing the volume of anesthesia and trauma, leading to a decrease in the rate of complications.

## Description

### FIELD OF THE INVENTION

The invention relates to the field of medicine and medical technology and is aimed at improving technical mesh endoprostheses for hernioplasty.

### STATE OF ART

A solution is known from the prior art (RU 121735 U1, publ. 10.11.2012), which describes a mesh implant for reconstructive surgery. The mesh implant has the form of a wire mesh made of a titanium alloy wire with a titanium content of at least 80%, wherein the wire mesh contains wires bent in the form of loops connected to each other. The wire mesh is fixed on the edges with interrupted, U-shaped sutures or continuous suture along the perimeter. If the most reliable fixation of the wire mesh to the tissues is needed, a second perimeter of the sutures can be applied along the edges of the hernial orifice. It is also possible to fix the wire mesh with titanium brackets.

A disadvantage of this solution is that when fixing the mesh implant, additional fixators are used in the form of sutures made in various ways or staples. This solution does not allow to establish quickly a strong fixation of the endoprosthesis and to ensure uniform fixation of the endoprosthesis with the entire surface thereof to the tissues of the surgical wound.

A solution is known from the prior art (RU 160627 U1, publ. 27.03.2016), which describes a mesh material for hernioplasty. A mesh made of titanium threads with a biologically active coating is fixed on the edges with interrupted, U-shaped sutures or a continuous suture along the perimeter. If the most reliable fixation of the mesh to the tissues is needed, a second perimeter of sutures can be applied along the edges of the hernial orifice.

A disadvantage of this solution, as well as the above one, is that when fixing the mesh implant, additional fixators are used in the form of sutures made in various ways or staples. This solution does not allow to establish quickly a strong fixation of the endoprosthesis and ensure uniform fixation of the endoprosthesis with the entire surface thereof to the tissues of the surgical wound; the threads do not have high plasticity and pose a high risk of breakage.

The claimed invention makes it possible to substantially overcome said disadvantages inherent in the prototype.

### DISCLOSURE OF THE INVENTION

The technical problem solved by the proposed technical solution is the development of a mesh titanium endoprosthesis for instant fixation in the operating field without additional fixators (suture material, staples, anchors, and glue).

The technical result consists in increasing the efficiency of fixing the endoprosthesis in the operating field, increasing the simplicity and strength of the fixation, ensuring the uniformity of fixing the endoprosthesis with the entire surface thereof to the tissues of the operating wound, increasing the plasticity and reducing the risk of breakage of the endoprosthesis threads, reducing the duration of surgery, and, as a consequence, reducing the volume of anesthesia and trauma leading to a decrease in the rate of complications and to a speedy recovery of the patient.

The technical result is achieved due to the fact that the self-fixing mesh endoprosthesis comprises or consists of two layers, upper and lower, with a layer of mesh based on titanium thread on the upper side, and a layer of bioresorbable polymers on the lower side, wherein the surface of the titanium thread is made in relief.

Titanium threads are made of GRADE-5 alloy.

The surface relief of the titanium thread is made with an uneven titanium thread diameter having fluctuations of from 0.00025 mm.

The surface of the relief titanium threads is coated with an oxide film.

A thickness of the titanium thread is 15-120 µm.

A surface density of the titanium mesh is 20-80 g/m².

A mesh size is 0.1-2 mm.

The lower layer is made of bioresorbable polymers selected from the group of polylactic acid.

Bioresorbable polymers are present in the form of drops with a diameter of 0.2-1 mm, located over the entire lower surface.

The lower layer of bioresorbable polymers contains at least one drug.

### BRIEF DESCRIPTION OF DRAWINGS

FIG. 1. A mesh endoprosthesis;
FIG. 2. Schematic representation of a cross-section of the endoprosthesis.
FIG. 3a. The state of the interloop range in a mesh fabric with relief threads.
FIG. 3b. The state of the interloop range in a mesh fabric with threads without a relief.
FIG. 4. Zero rigidity diagram for tensile of knitted meshes.
FIG. 5a. Example of the surface relief of a thread after chemical etching.
FIG. 5b. Example of the surface relief of a thread after ion treatment
FIG. 6a. Cross-section of a thread with longitudinal sharp-pointed defects before the treatment.
FIG. 6b. Cross-section of a thread with smoothed longitudinal defects after the treatment.

### IMPLEMENTATION OF THE INVENTION

The basis of the invention lies in the use of bioresorbable polymers as a fixator of a mesh endoprosthesis made of titanium thread.

The self-fixing mesh endoprosthesis 1 comprises or consists of two layers 2 and 3, wherein a layer 2 made of mesh based on titanium thread is on the upper side, and a layer 3 made of bioresorbable polymers is on the lower side.

The upper layer 2 is represented by filling-knit or warp-knitting mesh, it can be made of titanium thread from alloys VT1-00, VT-1.00 wa (GRADE-1) or VT6 (GRADE-5) with a thread thickness of 15-120 µm. The mesh has a surface density of 20-80 g/m², with a mesh size of 0.1-2 mm. The titanium threads used provide high biological inertness and plasticity of the threads, and make it possible to avoid tissue injury.

Titanium threads are made with a relief surface.

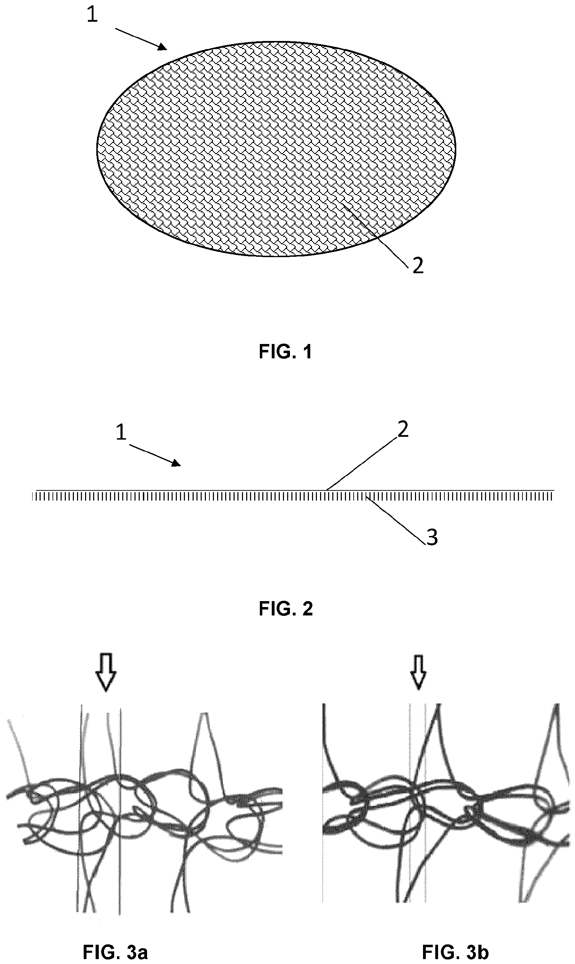

Technological processes making it possible to obtain a relief on the surface of a titanium thread are: power ultrasonic treatment, chemical etching, electrochemical polishing, ion treatment, etc. These methods of treatment reduce the diameter of the titanium thread by 10-35% of the initial diameter, at the same time reducing the area of interloop contacts. As a result, a "telescopic effect" is achieved: the penetration of loops and interloop floats into the area of adjacent loops, which is shown in FIG. 3a. This effect is not observed on the untreated thread (FIG. 3b). In Figures 3a and 3b, arrows and straight lines indicate the interpenetration of loops and interloop floats inside the loops in one looped wale, wherein interpenetration in FIG. 3a is much more.

The telescopic effect and reduction of resistance in the area of interloop contacts is the main factor in the elimination of "spring" properties. This fact is proved by measuring the mechanical properties of the material.

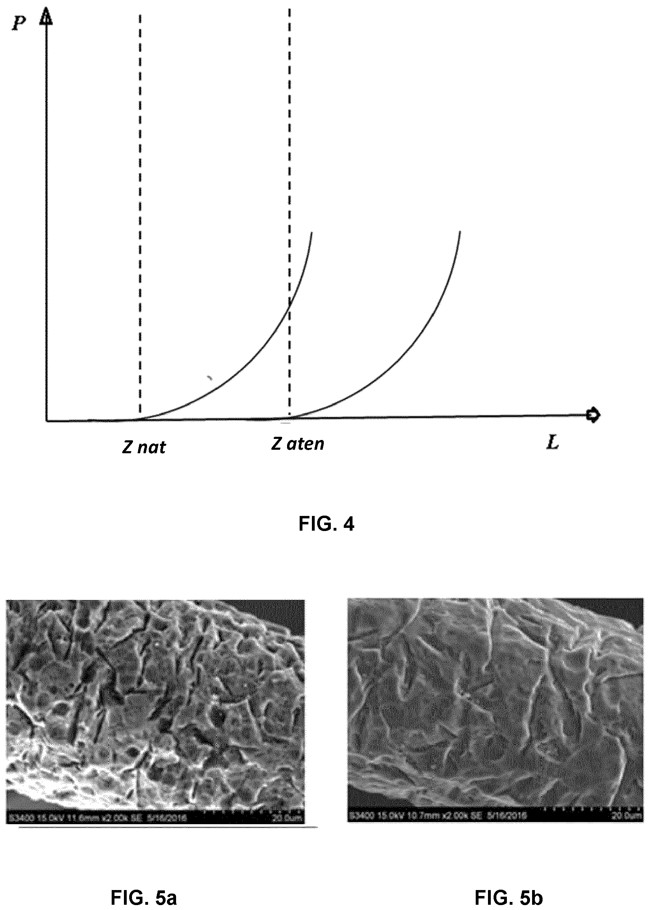

Therefore, when stretching knitted meshes, there is a period of zero rigidity Z, where Z aten is zero rigidity of the mesh fabric with relief threads (atension knitted metal fabric), and Z nat is the zero rigidity of the mesh fabric with threads without relief (native knitted metal fabric), that is the area on the diagram when the mesh fabric is stretched without resistance (FIG. 4). When comparing a conventional and treated mesh fabric of the same type of knitting and thread thickness, it is determined that the zone of zero stiffness of the treated mesh fabric made of relief threads is larger by 20% or more than that of an untreated one, with threads without a relief.

As a result of technological operations, a relief appears on the surface of the titanium thread: chaotically located depressions and bumps (FIG. 5a and 5b).

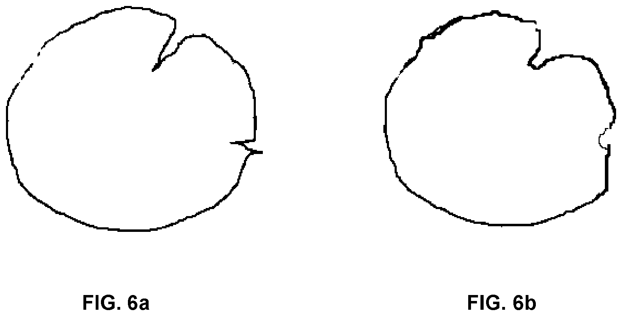

In addition, in the process of treatment on the surface of the thread located in the structure of the knitted mesh fabric, for example, by electrochemical polishing, longitudinal sharp-pointed defects (FIG. 6a) arising from the drawing of the thread are smoothed. The smoothing of defects after processing is shown in FIG. 6b. Smoothed longitudinal defects, which are the concentrators of internal stress, harmonize the residual stress in the thread itself and reduce the risk of the mesh fabric breakage.

A consequence of the treatment is also the unevenness of the titanium thread diameter with fluctuations along its length of from 0.00025 mm, which also provides additional freeness of interloop gaps.

To further increase the plasticity, an oxide film with a thickness of 1 to 3 µm, which has a low coefficient of sliding friction and allows the loops to easily slide relative to each other that positively affects the extensibility of the material, can be applied to the surface of relief titanium threads. The surface oxide film reduces friction between knitted loops and the accompanying negative properties: breakage when the material is straightened, etc. The oxide film is obtained by immersing mesh fabric made of relief threads into a galvanic bath filled with the necessary solution, with a constant current, for a certain time. Depending on the time and the selected voltage, an oxide film with a thickness of 1-3 µm is obtained on the surface of the titanium thread. In this case, the thickness of the thread itself does not increase.

The endoprosthesis 1 has an oval shape of 5-15 cm wide and the same length (FIG. 1).

Bioresorbable polymers are selected from the group of polylactic acid. They are applied as the lower (fixing) layer 3 (FIG. 2) by fixing to the lower pole of the mesh loop structure. The polymer layer can comprise or consist of droplets with a diameter of 0.2-1 mm. The drops are securely fixed, covering the mesh loops on three sides. The total thickness of the layer of drops does not exceed 0.2-2 mm. The thickness of the entire endoprosthesis is in the range of 0.5-4 mm.

Drugs can be incorporated into the structure of bioresorbable polymers: antibiotics, anti-inflammatory drugs, etc. Being gradually released, drugs can have a prolonged therapeutic effect, preventing inflammation and the development of wound infection.

The claimed invention makes it possible:
- to establish quickly a strong fixation of the endoprosthesis;
- to ensure uniform fixation of the endoprosthesis with the entire surface to the tissues of the surgical wound;
- significantly reduce the duration of surgery, and, as a result, the volume of anesthesia, less trauma, which leads to a decrease in the rate of complications;
- to create the possibility of temporary deposition of drugs gradually released from bioresorbable material;
- in the long term, the cleaning of the surgical wound from foreign bodies is ensured, with the exception of the titanium thread.

High plasticity of the mesh fabric minimizes spring properties, reduces the likelihood of biomechanical conflict between the tissue and the mucous membrane, and makes it possible to place the material under the mucous membrane without the risk of injury. The mesh endoprosthesis freely expands over the surface of the surgical wound, easily assumes and maintains a given shape, and is modeled according to the shape of the surgical wound by stretching, if necessary.

High porosity increases the rate of penetration of biological fluids into the endoprosthesis, accelerates the process of its colonization with fibroblasts and osteoblasts, and improves the biological integration of the material.

A mesh fabric made of titanium threads with a relief surface, being in contact with the wound surface, is instantly saturated with blood and wound discharge and exhibits pronounced adhesion to the wound surface, providing temporary self-fixation, allowing the surgeon to avoid using fixing elements: suture material, pins, micro-screws, etc. High adhesion to the wound surface allows the placement of a titanium mesh tensionless on the tissues underlying or covering the endoprosthesis, preventing such a frequent complication as surgical wound dehiscence.

At the same time, the highly porous structure does not retain the wound discharge, excluding the likelihood of fluid leaks and subsequent infection thereof.

The relief of the thread surface significantly improves the fixation of fibrin fibers thereon, thereby facilitating the attraction of fibroblasts serving as a source of newly formed connective tissue.

### Example 1

A model of a ventral hernia on the outer side of the anterior abdominal wall was obtained in three laboratory animals (rabbits, 4 months) through an open-cut surgery, and a self-fixing mesh implant 3x3 cm comprising or consisting of three monofilament threads with a diameter of 60 µm and a self-fixing layer 0.2 mm thick was installed. The implant was placed retroperitoneally without fixation with suture material. During the installation, high adhesion and instant fixation of the material to the bottom of the surgical wound were noted. The skin was sutured with interrupted sutures. Postoperative wound healing by primary intention. After 3 months, the animals were withdrawn from the experiment. When studying morphological changes, a whitish scar was found over the entire surface of the mesh implant, polylactic acid traces were not detected; upon microscopic examination, the structure of the postoperative scar was represented by ordered connective tissue fibers without signs of aseptic inflammation.

### Example 2

Interventions for implantation of the claimed endoprosthesis were performed in rats under general anesthesia with intraperitoneal Nembutal 30 mg/kg. The animals were 6 months old at the moment of the intervention.

The surgical wound was formed on the anterior abdominal wall of the laboratory animal by dissecting the skin, muscles, and subcutaneous adipose tissue as far as peritoneum. A self-fixing endoprosthesis was implanted. Skin was sutured. The wound was contaminated with a culture of *Staphylococcus aureus* 10⁹ CFU/ml. All animals were divided into 3 groups of 6 individuals each.

In the first group (control), meshes made of polypropylene thread (most often used in surgery) were used for implantation and sutured around the perimeter with polypropylene thread. In the second group, the first three individuals were implanted with a self-fixing endoprosthesis, with a titanium thread thickness of 15 µm, a surface density of the titanium thread of 20 g/m², a mesh size of 0.1 mm, bioresorbable polymer in the form of drops with a diameter of 0.2 mm, containing in addition to titanium mesh 35% (vol/vol) of polylactic acid and 3% of vancomycin; the second three individuals were implanted with a self-fixing endoprosthesis with a titanium thread thickness of 120 µm, a surface density of titanium thread of 80 g/m², a mesh size of 2 mm, bioresorbable polymer in the form of drops with a diameter of 1 mm, containing in addition to titanium mesh 35% (vol/vol) of polylactic acid and 3% of vancomycin; in the third group, the first three individuals were implanted with a self-fixing endoprosthesis, with a titanium thread thickness of 70 µm, a titanium thread surface density of 40 g/m², with a mesh size of 0.7 mm, a bioresorbable polymer in the form of drops with a diameter of 0.5 mm, containing in addition to the titanium mesh 35% (vol/vol) of polylactic acid and 5% of tobramycin; the second three individuals were implanted with the self-fixing endoprosthesis, with a titanium thread thickness of 100 µm, a surface density of titanium thread of 60 g/m², a mesh size of 1.5 mm, bioresorbable polymer in the form of drops with a diameter of 0.8 mm, containing in addition to the titanium mesh 35% (vol/vol) of polylactic acid and 5% of tobramycin. The observation lasted for 7 days.

In cases, a quick and easy installation of a self-fixing endoprosthesis was noted, which shortened the duration of surgery by about 15 minutes. It was noted that in the second and third groups of animals, the inflammation was localized. After installation, a firm uniform fixation of the endoprosthesis with the entire surface thereof to the tissues of the operating wound without displacement was observed, strong connective tissue formed in the area of implantation of the endoprosthesis was observed, toxic, allergic, and other side reactions were not observed. In the control group, infiltrative necrotic changes in the tissues of the surgical wound, adhesion formation, and uneven fixation of the endoprosthesis were observed, the installation process using additional fixators took more time than in the animals from the second and third groups. The most pronounced changes were observed at the latest dates of the study. Differences between the study groups and the control series were reliable throughout the whole observation period. Thus, the efficiency of using the claimed self-fixing endoprosthesis was noted.

The claimed self-fixing mesh endoprosthesis provides an increased efficiency of fixation in the operating field, increased plasticity and a low risk of endoprosthesis thread breakage; it is firmly and easily fixed, ensuring uniform fixation with the entire surface thereof to the tissues of the surgical wound, reducing the duration of surgery, and, as a consequence, reducing the volume of anesthesia and trauma, leading to reducing the rate of complications.

Further disclosed are the following aspects of the invention.
1. A self-fixing mesh endoprosthesis, comprising or consisting of two layers, upper and lower, with a layer of mesh based on titanium thread on the upper side, and a layer of bioresorbable polymers on the lower side, wherein the surface of the titanium thread is made in relief.
2. The self-fixing mesh endoprosthesis according to claim 1, wherein the titanium threads are made of GRADE-5 alloy.
3. The self-fixing mesh endoprosthesis according to claim 1, wherein the surface relief of the titanium thread is made with an uneven diameter of titanium thread having fluctuations of from 0.00025 mm.
4. The self-fixing mesh endoprosthesis according to claim 1, wherein an oxide film is applied to the surface of the relief titanium threads.
5. The self-fixing mesh endoprosthesis according to claim 1, wherein a thickness of the titanium thread is 15-120 µm.
6. The self-fixing mesh endoprosthesis according to claim 1, wherein a surface density of the titanium mesh is 20-80 g/m².
7. The self-fixing mesh endoprosthesis according to claim 1, wherein a mesh size is 0.1-2 mm.
8. The self-fixing mesh endoprosthesis according to claim 1, wherein the lower layer is made of bioresorbable polymers selected from the group of polylactic acid.
9. The self-fixing mesh endoprosthesis according to claim 1, wherein the bioresorbable polymers are present in the form of drops with a diameter of 0.2-1 mm located over the entire lower surface.
10. The self-fixing mesh endoprosthesis according to claim 1, wherein the lower layer of bioresorbable polymers contains at least one drug.

## Claims

1. A self-fixing mesh endoprosthesis, comprising two layers, upper and lower, with a layer of mesh based on titanium thread on the upper side, and a layer of bioresorbable polymers on the lower side, wherein the surface of the titanium thread is made in relief.

2. The self-fixing mesh endoprosthesis according to claim 1, wherein the titanium threads are made of GRADE-5 alloy.

3. The self-fixing mesh endoprosthesis according to claim 1, wherein the titanium threads are made of GRADE-1 alloy.

4. The self-fixing mesh endoprosthesis according to claim 1, wherein the surface relief of the titanium thread is made with an uneven diameter of titanium thread having fluctuations of from 0.00025 mm.

5. The self-fixing mesh endoprosthesis according to claim 1, wherein an oxide film is applied to the surface of the relief titanium threads.

6. The self-fixing mesh endoprosthesis according to claim 1, wherein a thickness of the titanium thread is 15-120 µm.

7. The self-fixing mesh endoprosthesis according to claim 1, wherein a surface density of the titanium mesh is 20-80 g/m².

8. The self-fixing mesh endoprosthesis according to claim 1, wherein a cell size is 0.1-2 mm.

9. The self-fixing mesh endoprosthesis according to claim 1, wherein the lower layer is made of bioresorbable polymers selected from the group of polylactic acid.

10. The self-fixing mesh endoprosthesis according to claim 1, wherein the bioresorbable polymers are present in the form of drops with a diameter of 0.2-1 mm located over the entire lower surface.

11. The self-fixing mesh endoprosthesis according to claim 1, wherein the lower layer of bioresorbable polymers contains at least one drug.

12. Use of bioresorbable polymers as a fixator of a mesh endoprosthesis made of titanium thread.
